# EUROPEAN PATENT SPECIFICATION

(11) **EP 1 216 036 B1**
(45) Date of publication and mention of the grant of the patent: **16.11.2005**
(21) Application number: 00964953.4
(22) Date of filing: 08.09.2000
(51) Int. Cl.: A61K 9/70, A61K 31/155

(54) **TRANSDERMAL ADMINISTRATION OF N-(2,5-DISUBSTITUTED PHENYL)-N'-(3-SUBSTITUTED PHENYL)-N'-METHYL GUANIDINES**
TRANSDERMALE VERABREICHUNG VON N-(2,5 DISUBSTITUIERTEN PHENYL)-N'-(3 SUBSTITUIERTEN PHENYL)-N'-METHYL GUANIDINEN
ADMINISTRATION TRANSCUTANEE DE N-(2,5-DISUBSTITUE PHENYL)-N'-(3-SUBSTITUE PHENYL)-N'-METHYL GUANIDINES

(30) Priority: 15.09.1999 US 153996 P
(43) Date of publication of application: 26.06.2002
(73) Proprietor: ALZA CORPORATION, Mountain View, CA 94039-7210 (US)
(72) Inventor: VAN OSDOL, William, W., Mountain View, CA 94040 (US); GALE, Robert, M., Los Altos, CA 94024 (US); BRANDWEIN, David, H., New Brighton, MN 55112 (US); PADMANABHAN, Rama, Los Altos, CA 94024 (US); SUNRAM, Joan, Coon Rapids, MN 55448 (US)
(74) Representative: Williams, Paul Edwin
(86) International application number: PCT/US2000/024682
(87) International publication number: WO 2001/019352

(56) References cited:
- WO-A-99/18962
- "Synthesis and Pharmacological Evaluation of N-(2,5-Disubstituted phenyl)-N'-(3-substituted phenyl)-N'-methylguanidines as N-Methyl-D-aspartate receptor ion-chanel blockers" J. MED. CHEM., 1997, pages 4281-4289, XP000926166 cited in the application & "Additions and corrections" J. MED. CHEM., vol. 41, 1998, page 1006
- LINDERS, JOANNES T. M.: "CNS - 5161 Cambridge NeuroScience Inc" CURR. OPIN. CENT. PERIPHER. NERV. SYST. INVEST. DRUGS (1999), 1(1), 167-170 , XP000982995
- BOKESCH, PAULA M. ET AL: "Neuroprotective, anesthetic, and cardiovascular effects of the NMDA antagonist, CNS 5161A, in isoflurane-anesthetized lambs" ANESTHESIOLOGY (2000), 93(1), 202-208 , XP000926176

## Description

### FIELD OF INVENTION

This invention relates to sustained release formulations for the safe and efficacious administration of N-(2,5-disubstituted phenyl)-N'-(3-substituted phenyl)-N'-methylguanidines as N-methyl-D-aspartate (NMDA) receptor ion-channel blockers. More particularly, the invention relates to devices for transdermally administering N-(2,5-disubstituted phenyl)-N'-(3-substituted phenyl)-N'-methylguanidines to a subject through a body surface or membrane over a sustained time period.

### BACKGROUND OF THE INVENTION

The transdermal route of parenteral delivery of drugs and other biologically active agents ("agents") has been proposed for a wide variety of systemically acting and locally acting agents on either a rate-controlled or non-rate-controlled basis and is described in numerous technical publications such as the following: U.S. Patent Nos. 3,598,122; 3,598,123; 3,731,683; 3,797,494; 4,031,894; 4,201,211; 4,286,592; 4,314,557; 4,379,454;. 4,435,180; 4,559,222; 4,573,995; 4,588,580; 4,645,502; 4,698,062; 4,704,282; 4,725,272; 4,781,924; 4,788,062; 4,816,258; 4,849,226; 4,904,475; 4,908,027; 4,917,895; 4,938,759; 4,943,435; 5,004,610; 5,071,656; 5,122,382; 5,141,750; 5,284,660; 5,314,694; 5,342,623.

When first investigated in depth in the late 1960's, the transdermal route of administration appeared to offer many advantages, particularly with respect to agents that had short half lives and therefore required frequent, repeated dosing or were subject to a high degree of first-pass metabolism by the liver. The peaks and valleys in blood concentration resulting from frequent periodic doses of short half-life agents would be eliminated and replaced by substantially constant plasma concentration. This would not only improve individual compliance but also would eliminate the alternating periods of high side-effects and ineffective blood concentrations associated with periodic dosing. Administering the agent through the skin directly into the blood stream would also eliminate first-pass metabolism of orally administered agents.

It was initially assumed, theoretically, that any short half-life agent of high potency and skin permeability would be suitable for safe and effective transdermal administration. This assumption, however, has not proven true.

The failure of the transdermal route to fulfill the initial expectations of its potential as an administrative portal was primarily due to the incredible variety of properties with which nature has endowed the skin to permit it to perform its function as the primary barrier to prevent the ingress of foreign substances into the body. See Transdermal Drug Delivery: Problems and Possibilities, B. M. Knepp, et al, CRC Critical Reviews and Therapeutic Drug Carrier Systems, Vol. 4, Issue 1 (1987) and Transdermal Delivery Systems: A Medical Rationale, Gary W. Cleary, Topical Drug Bioavailability, Bioequivalence, and Penetration, Plenum Press, 1993. Thus, the transdermal route of administration, rather than being available to every short half-life agent of high potency and skin permeability, was found to be available only to those few agents that possess the proper combination of a host of characteristics, most of which are unpredictable, required to render the agent suitable for safe and effective transdermal administration.

The most significant of these characteristics are the following:
1. Skin Permeability. The permeability of the skin.to the agent must be sufficiently high so that the agent can be administered at a therapeutically effective rate through an area of skin no greater than about 200 cm² and preferably no greater than 50 cm². The person-to-person variation in skin permeability at similar sites should also
   be considered. U.S. Patent Nos. 4,568,343; 4,746,515; 4,764,379; 4,863,738; 4,865,848; 4,888,354; 4,900,555; 5,378,730; 5,629,019; 5,641,504; 5,686,097; WO 95/09006; WO 95/01167; WO 96/37231, and WO 96/40259 are related to various compositions and methods for enhancing permeation of drugs through the skin:
2. Skin Binding. The skin beneath the transdermal delivery device has the capability of creating a skin depot of drug by absorbing, adsorbing, or binding a certain amount of agent. The amount of agent so bound must be supplied to the skin before the agent can be delivered into the blood stream at steady, therapeutically effective rates. If large amounts of the agent are bound in the skin, significant delays in the onset of therapeutic effect ("lag time") will be observed together with corresponding delays in termination of effect upon removal of the device. The potential also exists for toxic quantities of potent agents to be contained within the skin beneath the device. Skin binding is not related to skin permeability. Agents that are highly permeable may also be highly bound causing a lag time that is sufficiently long as to render them unsuitable for their intended use.
3. Irritation. The skin reacts to many topically applied substances, particularly those maintained under occlusion, by blistering or reddening accompanied by unpleasant burning, itching, and stinging sensations. Animal models are used to screen for irritation. Animal models, however, often produce both false positives and false negatives. There is also a wide interpersonal variation in susceptibility to irritation. An agent must be minimally irritating in a large percentage of the target population in order to be suitable for safe and effective transdermal administration. U.S. Patent Nos. 4,552,872; 4,756,710; 5,028,431; 5,130,139; 5,160,741; 5,304,379 and 5,451,407 are directed to overcoming problems of skin irritation associated with transdermal drug delivery.
4. Sensitization. Sensitization is an allergic reaction which is induced when an agent is first applied to the skin and is elicited upon continued exposure which may occur immediately or after a long period of seemingly harmless exposure.
   The sensitization may be local, elicited by topical exposure, which manifests itself as contact dermatitis accompanied by blistering, itching, reddening and buming at the site of application. More seriously, the sensitization may be systemic, elicited by topical application but manifesting itself by more general allergic reactions at sites other than the site of application. Most seriously, systemic sensitization may be elicited by oral or intravenous administration of the drug. If the latter occurs, the individual will be unable to take the drug by any route of administration.
   Animal models are used to screen for sensitization. Animal models, however, produce both false positives and false negatives. There is also a wide variation in the allergic reaction among individuals as well as between sexes, races and skin types. It is obvious that a useful transdermal agent must be minimally sensitizing in a large percentage of the target population. U.S. Patent Nos. 5,000,956; 5,049,387; 5,120,145 and 5,149,539 are directed to overcoming sensitization problems associated with transdermal drug delivery by the coadministration of a corticosteroid.
5. Pharmacokinetic Properties. The half-life of an agent is the length of time after administration that half of the amount administered has been eliminated from the body. Because blood concentrations of continuously administered agents continue to increase for approximately five half-lives before steady-state, constant blood concentrations are achieved, an agent must have a relatively short half-life to be suitable for continuous transdermal administration. The transdermal half-lives of most agents have not been determined. When half-lives of agents determined from intravenous administration are compared with half-lives determined from transdermal administration, the transdermal half-lives are generally longer but there can be wide variation in half-life between individuals based upon factors such as age, sex, health, and body type.
6. Pharmacodynamic Properties. Constant blood levels may not produce the desired therapeutic effects. For example, a therapeutic effect may only be observed at peak blood concentration obtained from bolus dosing but the peak blood or plasma concentration cannot be maintained because of side effects associated therewith. Also, continuous administration of many agents produces tolerance that may require either some agent-free interval or continually increasing the doses of agent, a potentially hazardous practice.
7. Potency. Although a certain degree of potency is required for transdermally administered agent to be effective, it is also possible for an agent to be too potent. As potency increases, lower blood concentrations are required and much smaller quantities are administered. Because of normal inter-individual variations in skin permeability and other factors, it may not be possible to precisely control whether a individual is receiving 1 µg/hr or 2 µg/hr, for example. For a highly potent agent, a 1 µg/hr administration may be totally ineffective and a 2 µg/hr rate may be fatal. Thus, the therapeutic index of an agent, which is the ratio of toxic blood concentration to the therapeutic blood concentration, becomes extremely significant. A highly potent agent should also have a relatively wide therapeutic window in order to be suitable for transdermal administration.
8. Metabolism. One of the perceived advantages of transdermal administration was that it avoided the "first-pass" metabolism of the agent by the liver that is associated with oral administration. It has now been recognized, however, that the skin is also a large metabolizing organ for some drugs. First-pass metabolism that occurs after an orally administered agent enters the blood stream can be avoided. However, skin metabolism, which occurs before the agent enters the bloodstream, cannot be avoided. Skin metabolism is capable of creating metabolites that are inactive, irritating, toxic, or comparable in biological activity to that of the agent. To be suitable for transdermal administration, an agent must have metabolic properties that are consistent with its therapeutic use on continuous administration.
   The above summarizes the primary characteristics that effect suitability of an agent for transdermal administration that have been recognized to date. There are undoubtedly others, some of which have not yet been recognized. In order for an agent to be suitable for transdermal administration, it must possess the right combination of all these characteristics. Such a combination, as illustrated by the very few drugs that are now suitable for administration from transdermal delivery devices, is quite rare and unpredictable.
   The present invention is directed to the transdermal administration of N-(2,5-disubstituted phenyl)-N'-(3-substituted phenyl)-N'-methyl guanidines. These compounds have been determined to be NMDA receptor ion-channel blockers, useful for the prevention of neuropathic pain, neuropsychological deficits resulting from cardiac surgery (CABG), and other neurological disorders. The inventors are unaware of this compound being administered transdermally by passive diffusion or electrically assisted transport across the skin.

### DESCRIPTION OF TERMS

As used herein, the term "guanidine" or "methyl guanidine" intends a compound selected from the group consisting of N-(2,5-disubstituted phenyl)-N'-(3-substituted phenyl)-N'-methyl guanidines and intends not only the basic form of the compound but also pharmaceutically acceptable salt forms, the R or S enantiomers, either individually or as a racemic mixture, and to mixtures thereof.

As used herein, the term "individual" intends a living mammal and includes, without limitation, humans and other primates, livestock and sports animals such as cattle, pigs and horses, and pets such as cats and dogs.

As used herein, the term "monoglyceride" refers to a monoglyceride or mixture of monoglycerides of C₈₋₂₀ fatty acids and includes, without limitation, glycerol monolaurate (GML), glycerol monooleate (GMO), glycerol monocaprate (GMC), glycerol monocaprylate (GMCL), and glycerol monolinoleate (GMLO).

As used herein, the term "permeation enhancement" intends an increase in the permeability of skin to the guanidine in the presence of a permeation enhancer as compared to permeability of skin to the guanidine in the absence of a permeation enhancer.

As used herein, the term "permeation enhancer" intends a compound or a mixture of compounds which acts to increase the permeability of the skin to a guanidine.

As used herein, the term "permeation-enhancing amount" intends an amount of a permeation enhancer which provides permeation enhancement throughout a substantial portion of the administration period.

As used herein, the phrase "predetermined area of skin" intends a defined area of intact unbroken skin or mucosal tissue. That area will usually be in the range of about 5 cm² to about 100 cm².

As used herein the term "salt" intends, but is not limited to, pharmaceutically acceptable organic or inorganic salts. Typical inorganic salts include hydrogen halides such as hydrochlorides, carbonates, phosphates, sulfates, hydrogen sulfates, hydrobromides, nitrates, and sulfides. Organic salts include, but are not limited to, acid addition salts including salts of monocarboxylic and polycarboxylic acids such as acetic acid, malic acid, maleic acid, propionic acid, succinic acid, fumaric acid, citric acid, benzoic acid, cinnamic acid, tartaric acid, and the like.

As used herein, the phrase "sustained time period" or "administration period" intends a period of at least about 8 hours and will typically intend a period in the range of about one to about seven days.

As used herein, the term "therapeutically effective amount" intends the dose of N-(2,5-disubstituted phenyl)-N'-(3-substituted phenyl)-N'-methylguanidine and/or its active metabolites that provides effective therapy for the treatment of, among others, neuropathic pain, neuropsychological deficits resulting from cardiac surgery (CABG), and other neurological disorders. In the case of adult and juvenile humans, the dosage range is about 0.1 -10 mg/day.

As used herein, the term "therapeutically effective rate" intends a delivery rate of N-(2,5-disubstituted phenyl)-N'-(3-substituted phenyl)-N'-methylguanidines and/or its active metabolites effective to achieve therapeutic blood or plasma levels in an individual during the administration period and is typically within the range of about 1 - 420 µg/hr.

As used herein, the term "therapeutic blood or plasma level" intends the level of N-(2,5-disubstituted phenyl)-N'-(3-substituted phenyl)-N'-methyl guanidines and/or its active metabolites in blood or plasma that achieves a therapeutic effect for the desired therapy.

As used herein, the term "transdermal" intends both percutaneous and transmucosal administration, i.e., passage of guanidine through a body surface or membrane such as intact unbroken skin or mucosal tissue into the systemic circulation.

### SUMMARY OF THE INVENTION

This invention provides delivery systems for effecting transdermal delivery of a guanidine and/or its active metabolites, which are suitable for the transdermal administration of the compounds continuously through a body surface or membrane at a therapeutically effective rate in order to achieve and maintain therapeutic blood or plasma levels in an individual.

According to this invention, it has been discovered that guanidines can be safely and efficaciously administered transdermally at a therapeutically effective rate to prevent, among other things, neuropathic pain, neuropsychological deficits resulting from cardiac surgery (CABG), and other neurological disorders. The preparation of the compounds for transdermal administration according to this invention are described in Lain-Yen Hu et al. "Synthesis and Pharmacological Evaluation of N-(2,5-disubstituted phenyl)-N'-(3-substituted phenyl)-N'-methylguanidii1es as N-Methyt - D- Aspartate Receptor Ion-Channel Blockers". J. Med. Chem, vol 40, pgs. 4281-4289 (1997).

Pharmaceutical compositions comprising N-(2-chloro-5-methylthiophenyl)-N'-(3-methylsulfinylphenyl)-N'-methylguanidine or pharmaceutically acceptable salts thereof are know from WO 99/18962. Therefore, the invention comprises the following aspect.

A device for the transdermal administration of a guanidine compound at a therapeutically effective rate according to this invention comprises:
(a) a reservoir comprising a guanidine and, optionally, a permeation-enhancing amount of a permeation enhancer,
(b) a backing behind the body contacting-distal surface of the reservoir; and
(c) means for maintaining the reservoir in drug transmitting relation with a body surface or membrane, wherein a therapeutically effective amount of a guanidine is delivered at a therapeutically effective rate during an administration period in order to achieve and maintain therapeutic blood or plasma levels throughout a substantial portion of the administration period.

The permeation enhancer may be any permeation enhancer known in the art to increase permeability of drugs through skin and includes, but is not limited to, those disclosed in the above cited patents. Preferably, the permeation enhancer comprises a permeation enhancing amount of a permeation enhancer including, but not limited to pyroglutamic acid esters such as lauryl pyroglutamate, monoglycerides, C₁₀-C₂₀ fatty acid esters including ethyl palmitate and isopropyl myristate; acyl lactylates such as caproyl lactylic acid and lauroyl lactylic acid; dimethyl lauramide; dodecyl (lauryl) acetate; lactate esters such as lauryl lactate, and myristyl lactate; monoalkyl ethers of polyethyleneglycol and their alkyl or aryl carboxylic acid esters and carboxymethyl ethers such as polyethylene glycol-4 lauryl ether (Laureth-4) and polyethylene glycol-2 lauryl ether (Laureth-2); Myreth-3, myristyl sarcosine, and methyl laurate.

These and other aspects of the present invention will be readily apparent from the description and accompanying figures that follow.

### BRIEF DESCRIPTION OF THE FIGURES

Figure 1 is a cross-section through a schematic perspective view of one embodiment of a transdermal therapeutic system according to this invention.
Figure 2 is a cross-section view through another embodiment of this invention prior to application to the skin.
Figure 3 is a cross-section view through another embodiment of this invention prior to application to the skin.
Figure 4 is a cross-section view through another embodiment of this invention prior to application to the skin.
Figure 5 depicts the passive transdermal flux of CNS 5161 from several matrix devices.
Figure 6 depicts the transdermal flux of CNS 5161 by electrotransport.

### DETAILED DESCRIPTION OF THE INVENTION

According to this invention, it has been found that the guanidine compounds according to this invention can be safely and efficaciously administered transdermally at a therapeutically effective rate to provide, among other things, treatment for neuropathic pain, neuropsychological deficits resulting from cardiac surgery (CABG), and other neurological disorders. The present invention provides devices for guanidine therapy with improved patient compliance to an individual in need of such therapy.

According to this invention, the target therapeutic dose is about 0.1 - 10 mg/day, preferably 0.1 - 2 mg/day, and most preferably 0.25 - 1 mg/day. Therapeutic blood or plasma levels can be obtained from transdermal administration rates in the range of 1 - 420 µg/hr, preferably about 4 - 100 µg/hr, and most preferably 10 - 50 µg/hr. Representative steady-state *in vitro* fluxes of the guanidine through human skin by passive diffusion are in the range of about 0.1 - 2 µg/cm²hr, depending on the drug form, permeation enhancer, and adhesive. Electrically assisted transport of the guanidine through skin resulted in an average steady-state transdermal flux of about 50 - 60 µg/cm²hr at a current of 0.2mA and current density of 0.1 mA/cm².

This invention finds particular usefulness in administering guanidines across skin. It is also useful, however, in administering the compounds across mucosa. According to the invention, guanidine is placed in guanidine transmitting relationship to an appropriate body surface, preferably in a pharmaceutically acceptable carrier thereof, and maintained in place for the desired administration period.

The N-(2,5-disubstituted phenyl)-N'-(3-substituted phenyl)-N'-methylguanidines suitable for use according to this invention and their synthesis are disclosed in the Hu et al. article listed above. Preferred guanidines according to this invention include N-(2-chloro-5-(methylthio)phenyl)-N'-(3-(methylthio)phenyl)-N'-methyl guanidine and N-(2-bromo-5-(methylthio)phenyl)-N'-(3-(methylthio)phenyl)-N'-methyl guanidine.

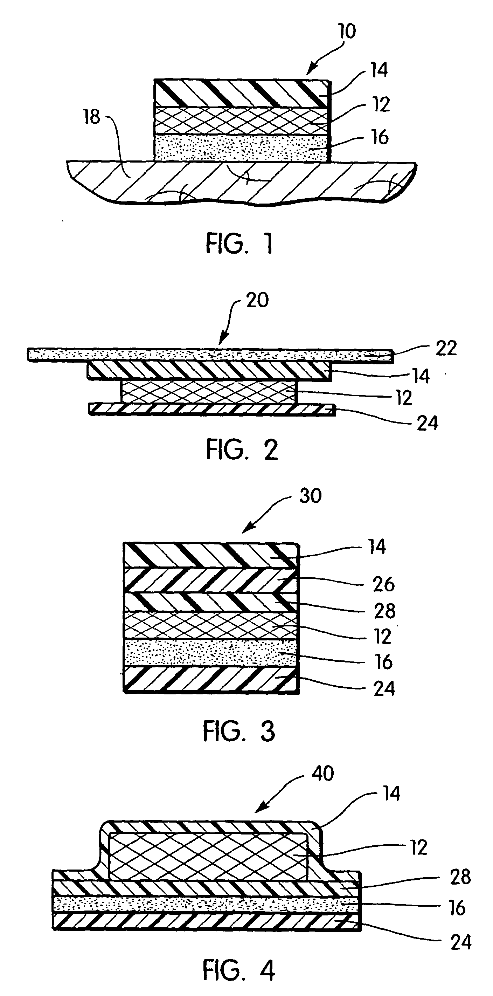

A guanidine according to this invention is administered from a transdermal delivery device as more fully described below. Examples of suitable transdermal delivery devices are illustrated in Figs. 1-4. In the figures, the same reference numbers are used throughout the different figures to designate the same or similar components. The figures are not drawn to scale.

Referring now to Figure 1, one embodiment of a transdermal therapeutic system according to this invention comprises transdermal delivery device 10 comprising a reservoir 12, preferably in the form of a matrix containing guanidine and optionally, a permeation enhancer dispersed therein. Reservoir 12 is sandwiched between a backing 14 and an in-line contact adhesive layer 16. The device 10 adheres to the surface of the skin 18 by means of the adhesive layer 16. The adhesive layer 16 may optionally contain the permeation enhancer and/or guanidine. A removable release liner (not shown in FIG. 1) is normally provided along the exposed surface of adhesive layer 16 and is removed prior to application of device 10 to the skin 18. Optionally, a rate-controlling membrane (not shown) may be present between the reservoir 12 and the adhesive layer 16. Additionally, a non-rate controlling tie layer membrane, as disclosed in US Patent No. 5,635,203, may be present between the reservoir 12 and adhesive 16 in any of the embodiments depicted in Figures 1 - 4.

Although the depicted embodiments of this invention utilize an in-line adhesive as shown in Figure 1, other means for maintaining the system on the skin can be employed. Such means include a peripheral ring of adhesive outside the path of the drug from the system to the skin or the use of other fastening means such as buckles, belts, and elastic arm bands.

According to a preferred embodiment, reservoir 12 may be in the form of a matrix containing a guanidine and, optionally, a permeation enhancer dispersed within a suitable adhesive, preferably a pressure sensitive adhesive. Such pressure sensitive adhesives include, but are not limited to, polysiloxanes, polyacrylates, polyurethanes, acrylic adhesives including cross linked or non-crosslinked acrylic copolymers, vinyl acetate adhesives, ethylene vinylacetate copolymers, and natural or synthetic rubbers including polybutadienes, polyisoprenes, and polyisobutylene adhesives, and mixtures and graft copolymers thereof.

The matrix formulations according to this embodiment comprise the adhesive containing the guanidine and permeation enhancer, if present, laminated to a backing on one surface and to a release liner on the other. In addition to the guanidine and permeation enhancer, the matrix or carrier may also contain dyes, pigments, inert fillers, anti-irritants, excipients, preservatives (for example antioxidants) and other conventional components of pharmaceutical products or transdermal devices known to the art. For example, the matrix may also be provided with hydrophilic water absorbing and water soluble polymers known in the art such as polyvinyl alcohol and polyvinyl pyrrolidone individually or in combination. Other suitable water soluble and water absorbing polymers are known in the art, such as those disclosed in U.S. Patent No. 5,176,916.

Altematively, as shown in FIG. 2, transdermal therapeutic device 20 may be attached to the skin or mucosa of a patient by means of an adhesive overlay 22. Device 20 is comprised of reservoir 12 preferably in the form of a matrix containing guanidine and, optionally, a permeation enhancer dissolved and/or dispersed therein. A backing layer 14 is provided adjacent to one surface of reservoir 12. Adhesive overlay 22 maintains the device on the skin and may be fabricated together with, or provided separately from, the remaining elements of the device. With certain formulations, the adhesive overlay 22 may be preferable to the in-line contact adhesive 16 as shown in FIG. 1. Backing layer 14 is preferably slightly larger than reservoir 12, and in this manner prevents the materials in reservoir 12 from adversely interacting with the adhesive in overlay 22. Optionally, a rate-controlling membrane (not shown in FIG. 2) may be provided on the skin-proximal side of reservoir 12. A removable release liner 24 is also provided with device 20 and is removed just prior to application of device 20 to the skin.

In FIG. 3, transdermal delivery device 30 comprises a guanidine and permeation enhancer reservoir ("guanidine reservoir") 12 substantially as described with respect to FIG. 1. Permeation enhancer reservoir ("enhancer reservoir") 26 comprises the permeation enhancer dispersed throughout and contains a guanidine at or below saturation, when in equilibrium with the guanidine reservoir 12. Enhancer reservoir 26 is preferably made from substantial the same matrix as is used to form guanidine reservoir 12. A rate-controlling membrane 28 for controlling the release rate of the permeation enhancer from enhancer reservoir 26 to guanidine reservoir 12 is placed between the two reservoirs. A rate-controlling membrane (not shown in FIG. 3) for controlling the release rate of the enhancer and/or guanidine from guanidine reservoir 12 to the skin may also optionally be utilized and would be present between adhesive layer 16 and reservoir 12.

The rate-controlling membrane may be fabricated from permeable, semipermeable or microporous materials which are known in the art to control the rate of agents into and out of delivery devices and having a permeability to the permeation enhancer lower than that of drug reservoir 12. Suitable materials include, but are not limited to, polyethylene, polyvinyl acetate, ethylene n-butyl acetate and ethylene vinyl acetate copolymers.

Superimposed over the permeation enhancer reservoir 26 of device 30 is a backing 14. On the skin-proximal side of reservoir 12 are an adhesive layer 16 and a removable liner 24 which would be removed prior to application of the device 30 to the skin.

In the embodiments of FIGS. 1, 2 and 3, the carrier or matrix material of the reservoirs has sufficient viscosity to maintain its shape without oozing or flowing. If, however, the matrix or carrier is a low-viscosity flowable material such as a liquid or a gel, the composition can be fully enclosed in a pouch or pocket, as known to the art from US Pat. No. 4,379,454 (noted above), for example, and as illustrated in FIG. 4.

Device 40 shown in FIG. 4 comprises a backing member 14 which serves as a protective cover for the device, imparts structural support, and substantially keeps components in device 40 from escaping the device. Device 40 also includes reservoir 12, which contains the guanidine and permeation enhancer and bears on its surface distant from backing member 14, a rate-controlling membrane 28 for controlling the release of guanidine and/or permeation enhancer from device 40. The outer edges of backing member 14 overlay the edges of reservoir 12 and are joined along the perimeter with the outer edges of the rate-controlling membrane 28 in a fluid-tight arrangement. This sealed reservoir may be effected by pressure, fusion, adhesion, an adhesive applied to the edges, or other methods known in the art. In this manner, reservoir 12 is contained wholly between backing member 14 and rate-controlling membrane 28. On the skin-proximal side of rate-controlling membrane 28 are an adhesive layer 16 and a removable liner 24 which would be removed prior to application of the device 40 to the skin.

In an alternative embodiment of device 40 of FIG. 4, reservoir 12 contains the permeation enhancer and contains guanidine at or below saturation. The guanidine and an additional amount of permeation enhancer are present in adhesive layer 16, which acts as a separate reservoir.

The guanidine can be administered to human skin or mucosa by direct application to the skin or mucosa in the form of an ointment, gel, cream or lotion, for example, but are preferably administered from a skin patch or other known transdermal delivery device which contains a saturated or subsaturated formulation of the guanidine and optional enhancer. The formulation may be aqueous or non-aqueous. The formulation should be designed to deliver the guanidine and any anti-irritant and/or enhancer at the necessary fluxes. Aqueous formulations typically comprise water or water/ethanol and about 1-5 wt% of a gelling agent, an example being a hydrophilic polymer such as hydroxyethylcellulose or hydroxypropylcellulose. Typical non-aqueous gels are comprised of silicone fluid or mineral oil. Mineral oil-based gels also typically contain 1-2 wt% of a gelling agent such as colloidal silicon dioxide. The suitability of a particular gel depends upon the compatibility of its constituents with the guanidine, anti-irritant, and the permeation enhancer in addition to any other components in the formulation.

The reservoir matrix should be compatible with the guanidine, the permeation enhancer, and any carrier therefor. The term "matrix" as used herein refers to a well-mixed composite of ingredients. When using an aqueous formulation, the reservoir matrix is preferably a hydrophilic polymer, e.g., a hydrogel.

When using a non-aqueous formulation, the reservoir matrix is preferably composed of a hydrophobic polymer. Suitable polymeric matrices are well known in the transdermal drug delivery art, and examples include polyurethanes, as well as the materials listed in the above-named patents.

A typical laminated system would consist essentially of a polymeric membrane and/or matrix such as ethylene vinyl acetate (EVA) copolymers, such as those described in US Pat. No. 4,144,317, preferably having a vinyl acetate (VA) content in the range of from about 9% up to about 60% and more preferably about 9% to 40% VA. Polyisobutylene/oil polymers containing from 4-25% high molecular weight polyisobutylene and 20-81% low molecular weight polyisobutylene with the balance being an oil such as mineral oil or polybutene may also be used as the matrix material. According to the preferred embodiment, the polymeric matrix comprises a pressure sensitive adhesive as listed above.

The amount of the guanidine present in the therapeutic device and required to achieve an effective therapeutic result depends on many factors, such as the minimum necessary dosage of the guanidine for the particular indication being treated; the solubility and permeability of the matrix, taking into account the presence of permeation enhancer, of the adhesive layer and of the rate-controlling membrane, if present; and the period of time for which the device will be fixed to the skin. The minimum amount of guanidine is determined by the requirement that sufficient quantities of guanidine must be present in the device to maintain the desired rate of release over the given period of application. The maximum amount for safety purposes is determined by the requirement that the quantity of guanidine present must not support a rate of release that reaches toxic levels.

The guanidine may be present in the matrix or carrier at a concentration at or below saturation. An excess amount of the guanidine above saturation may be included in the matrix or carrier, the amount of excess being a function of the desired length of the delivery period of the system. The guanidine may be present at a level below saturation without departing from this invention as long as it is continuously administered to the skin or mucosal site at a therapeutic rate and for a period of time sufficient to deliver a therapeutically effective amount of guanidine that provides the desired therapeutic result.

The permeation enhancer useful in the present invention is selected from those compounds which are compatible with the guanidine compound and which provide enhanced skin permeation to the drug when it is administered together with the drug to the skin of a user. Additionally, the permeation enhancer must not adversely interact with the adhesive of the in-line contact adhesive layer if one is present. Examples of permeation enhancers are disclosed in the patents cited above and can be selected from, but are not limited to, lauryl pyroglutamate, fatty acids, monoglycerides of fatty acids such as glycerol monolaurate, glycerol monooleate, glycerol monocaprate, glycerol monocaprylate, or glycerol monolinoleate; lactate esters of fatty acids such as lauryl lactate, cetyl lactate, and myristyl lactate; acyl lactylates such as caproyl lactylic acid; esters of fatty acids having from about 10 to about 20 carbon atoms, including, but not limited to, isopropyl myristate, and ethyl palmitate; alkyl laurates such as methyl laurate; dimethyl lauramide; lauryl acetate; monoalkyl ethers of polyethyleneglycol and their alkyl or aryl carboxylic acid esters and carboxymethyl ethers such as polyethylene glycol-4 lauryl ether (Laureth-4) and polyethylene glycol-2 lauryl ether (Laureth-2); polyethylene glycol monolaurate; myristyl sarcosine; Myreth-3; and lower C₁₋₄ alcohols such as isopropanol and ethanol, alone or in combinations of one or more. A preferred permeation enhancer according to this invention comprises lauryl pyroglutamate.

The permeation-enhancing mixture is dissolved and/or dispersed through the matrix or carrier, preferably at a concentration sufficient to provide permeation-enhancing amounts of enhancer in the reservoir throughout the anticipated administration period. Where there is an additional, separate permeation enhancer matrix layer as well, as in FIGS. 3 and 4, the permeation enhancer normally is present in the separate reservoir in excess of saturation.

The devices according to this invention may also contain an anti-irritant dispersed throughout the matrix or carrier, preferably at a concentration sufficient to deliver anti-irritant to the skin in an amount effective to reduce skin irritation throughout the anticipated administration period. The anti-irritant is preferably present in excess of saturation in order to ensure that the anti-irritant is continuously administered with the guanidine and continues to be present as long as any guanidine is present in the epidermis. Suitable anti-irritants include, but are not limited to, methyl nicotinate as disclosed in US Patent No. 5,451,407, corticosteroids, and buffering agents including ascorbic acid and acetic acid. Such anti-irritants are known in the art as seen in the above cited patents.

Because of the wide variation in skin permeability from individual to individual and from site to site on the same body, it may be preferable that the guanidine, anti-irritant, and/or permeation enhancer, be administered from a rate-controlled transdermal delivery device. Rate control can be obtained either through a rate-controlling membrane as described in U.S. Patent No. 3,797,494 listed above, or through an adhesive or both as well as through other means known in the art.

A certain amount of the guanidine may bind reversibly to the skin, and it is accordingly preferred that the skin-contacting layer of the device include this amount of the guanidine as a loading dose.

The surface area of the device of this invention can vary from about 1-200 cm². A typical device, however, will have a surface area within the range of about 5-60 cm², preferably less than about 20 cm².

The devices of this invention can be designed to deliver the guanidine effectively for an extended time period of from several hours up to 7 days or longer. Seven days is generally the maximum time limit for application of a single device because the adverse effects of occlusion of a skin site increase with time and the normal cycle of sloughing and replacement of the skin cells occurs in about 7 days.

Preferably, a device for the transdermal administration of a guanidine, at a therapeutically effective rate, comprises:
(a) a reservoir comprising:
   (i) 1-30% by weight guanidine,
   (ii) 0-50% by weight of a permeation enhancer,
   (iii) 30 to 90% by weight of a polymeric carrier;
(b) a backing behind the skin-distal surface of the reservoir; and
(c) means for maintaining the reservoir in guanidine - transmitting relation with the skin.

More preferably, a device for the transdermal administration of a guanidine, at a therapeutically effective rate, comprises:
(a) a reservoir comprising:
   (i) 1 - 25% by weight guanidine,
   (ii) 1 - 30% by weight of a permeation enhancer,
   (iii) 30 - 90% by weight of a polymeric carrier;
(b) a backing behind the skin-distal surface of the reservoir; and
(c) means for maintaining the reservoir in guanidine- transmitting relation with the skin.

Most preferably, a device for the transdermal administration of a guanidine, at a therapeutically effective rate, comprises a matrix system comprising:
(a) a reservoir comprising:
   (i) 1 - 15% by weight N-(2-Chloro-5-(methylthio)phenyl)-N'-(3-(methylthio)phenyl)-N'-methyl guanidine
   (ii) 4 - 20% by weight lauryl pyroglutamate
   (iii) 30 - 90% by weight of a polymeric carrier;
(b) a backing behind the skin-distal surface of the reservoir; and
(c) means for maintaining the reservoir in guanidine - transmitting relation with the skin.

The backing may be flexible or nonflexible and may be a breathable or occlusive material. Suitable materials include, without limitation, polyethylene, polyurethane, polyester, ethylene vinyl acetate, acrylonitrile, cellophane, cellulose acetate, cellulosics, ethylcellulose, ethylene vinyl alcohol, plasticized vinylacetate-vinylchloride copolymers, polyethylene terephthalate, nylons, rayon, polypropylene, polyvinyl alcohol, polyvinyl chloride, metalized polyester films, polyvinylidene chloride, polycarbonate, polystyrene, and aluminum foil. The backing may be a multi-laminate film.

The means for maintaining the reservoir in drug and permeation enhancer transmitting relation with the skin is preferably a pressure sensitive adhesive including, but not limited to, polyisobutylene adhesives, silicone adhesives, and acrylate adhesives known in the art induding copolymers and graft copolymers thereof. A further embodiment of the invention is directed to including in the adhesive a small percentage, e.g., from about 1 to about 5 wt% of guanidine to assure an appropriate initial release rate. The means for maintaining may also double as the reservoir, thereby providing a drug-in-adhesive monolithic formulation.

About 0.1-10 mg/day, preferably 0.1 - 2 mg/day, and most preferably 0.25 -1 mg/day of the guanidine are delivered daily by the devices disclosed above. To achieve this result, guanidine is delivered at a therapeutic rate within a range of about 1-420 µg/hr, preferably about 4-100 µg/hr, most preferably 10-50 µg/hr from a reasonably sized transdermal delivery device having a surface area of less than about 60 cm², preferably less than about 20 cm², for the treatment period, usually about 12 hours to 5 days, preferably 24 - 72 hours.

The length of time of guanidine presence and the total amount of guanidine in the plasma can be changed following the teachings of this invention to provide different treatment regimens. Thus, they can be controlled by the amount of time during which exogenous guanidine is delivered transdermally to an individual or animal and the rate at which it is administered.

Having thus generally described our invention, the following specific examples describe preferred embodiments thereof.

### EXAMPLE 1

### Fabrication of Transdermal Adhesive Matrix Systems

Using sonication, 2.75 g of N-(2-chloro-5-(methylthio)phenyl)-N'-(3-(methylthio)phenyl)-N'-methyl guanidine hydrochloride (CNS 5161A HCl provided by Cambridge NeuroScience, Cambridge MA) were dissolved in Milli-Q water at 11 mg/mL. The solution was titrated with ammonium hydroxide to achieve 100% conversion (final pH 9.2 following addition of 1.9 equivalents of ammonium hydroxide). The drug base was extracted into chloroform in a seperatory funnel. Three aliquots of the chloroform layer were dried with nitrogen gas, reconstituted in mobile phase and the drug content was verified by HPLC. The yield of drug base was 98.2% of the theoretical value. Measured volumes of the chloroform layer were delivered into tubes and dried using a stream of warm nitrogen gas. The drug base, a light brown gel, was further dried under vacuum for 6.5 hours. The tubes were tightly capped, protected from light and stored at 4°C.

The following six formulations, three containing the base and three the HCl salt were configured for assessment of transdermal permeation. NS 87-2287 is a polyacrylate adhesive provided by National Starch and Chemical Company.
*1) Formulation A:* 6% CNS 5161 (free base), NS 87-2287 polyacrylate adhesive
*2) Formulation B:* 6% CNS 5161 (free base), 12% permeation enhancer lauryl pyroglutamate, NS 87-2287 polyacrylate adhesive
*3) Formulation C:* 6% 5161 (free base) 3% glycerol monolaurate, NS 87-2287 polyacrylate adhesive
*4) Formulation D:* 6% CNS 5161A (HCl salt), NS 87-2287 polyacrylate adhesive
*5) Formulation E:* 6% CNS 5161A (HCl salt), 12% lauryl pyroglutamate, NS 87-2287 polyacrylate adhesive
*6) Formulation F:* 6% CNS 5161A (HCl salt), 3% glycerol monolaurate, NS 87-2287 polyacrylate adhesive

For formulations containing the drug base, the base was dissolved in ethyl acetate. The resulting solution of known drug concentration was used in the formulation in appropriate amounts. The drug HCl salt powder was weighed out directly. The formulation mixtures comprising the drug base/salt, adhesive, permeation enhancer and ethyl acetate were then thoroughly mixed.

### Procedure to Hand-Cast Adhesive Matrix Systems

Glass plates cleaned with isopropyl alcohol were used as supports to hand-cast the formulations. Appropriate lengths of siliconized release liner (release side up) were smoothed onto the plates and secured. A puddle of the formulation mixture was spread across one end of the liner and with a casting knife was smoothly drawn down the length of the liner. After drying in a fume hood overnight the casts were further dried in an oven at 65°C for a minimum of 1 hour. The dried matrices were covered with siliconized release liner (release side down) and calendered to a required thickness of 2 mils. After removing one of the liners, a backing film was hand- laminated to each cast. Individual systems of appropriate areas were punched out from the casts prior to use.

Heat-separated human epidermis from several skin donors and anatomic sites was used for the in vitro permeation studies. The epidermis was separated from the dermis following immersion in 60°C water for 60 seconds. The epidermis was screened for defects prior to use.

A 2 cm² system was centered and attached to the epidermal side of a 4.5 cm² piece of epidermis. Two layers of double-stick tape rings were placed on the skin. The epidermis with the attached system was centered over the opening of the receptor compartment and the skin was secured to the cell with the double-stick tape rings. A foam cushion and a Delrin end piece were added and secured.

Cell and tubing materials were chosen on the basis of drug compatibility. The selected receptor solution (citrate (10 mM) buffered saline (150 mM), pH 5.25) and the permeation cells were maintained at 32°C.

### Sampling Procedure

The entire receptor volume of the permeation cell was automatically collected and refilled every 2 hours for a total of 72 hours. Samples were collected into tared test tubes, which were weighed again to determine sample volume. Drug content of selected receptor samples was determined by HPLC. Typically, all samples collected during the first 24 hours and alternate samples thereafter were analyzed by HPLC.

### Calculation of Transdermal Flux

Flux was calculated from the receptor sample volume multiplied by the sample concentration and divided by the sample interval (typically 2 hours) and the skin contact area (typically 2 cm²). Transdermal flux (µg/cm²h) was plotted versus time to obtain a flux profile. Average steady-state flux values were calculated typically including samples collected between 12 to 72 hours for formulations with the drug base and 20 to 72 hours for formulations with the drug salt.

### Drug Analysis by HPLC

Solvent A comprised of 5 / 95 / 0.1, ACN / water / TFA while solvent B was 95/5/0.1, ACN / water / TFA. The gradient was from 100% A to 100% B at 1.0 mL/min flow rate, recording absorbance at 220 nm using a Beckman Ultrasphere C-18 column (5 µm, 4.6 x 250 mm) with a 25 µL injection at room temperature.

An isocratic assay, based on the elution conditions of the gradient assay, was developed to facilitate quick analysis of receptor samples generated during the flux experiments. Solvent conditions were at 50% solvent B. All other conditions were the same as above. The run time was 8 minutes and separations exhibited 5700 theoretical plates at 100 µg free base equivalents (FBE)/mL and 9770 theoretical plates at 16 µg FBE/mL.

### Drug Release Rates

Matrix systems A - F (2 cm² area) were placed in 15 mL polypropylene bottles each containing 12 mL of receptor solution (10 mM citrate buffered saline containing 150 mM NaCl, pH 5.25). The bottles were incubated on a lateral shaker at room temperature. Aliquots of receptor solution were sampled over time and analyzed for drug by HPLC. Cumulative drug release (as µg/cm²) from each system into the citrate buffered saline over 24 hours was determined.

### In Vitro Transdermal Passive Drug Flux

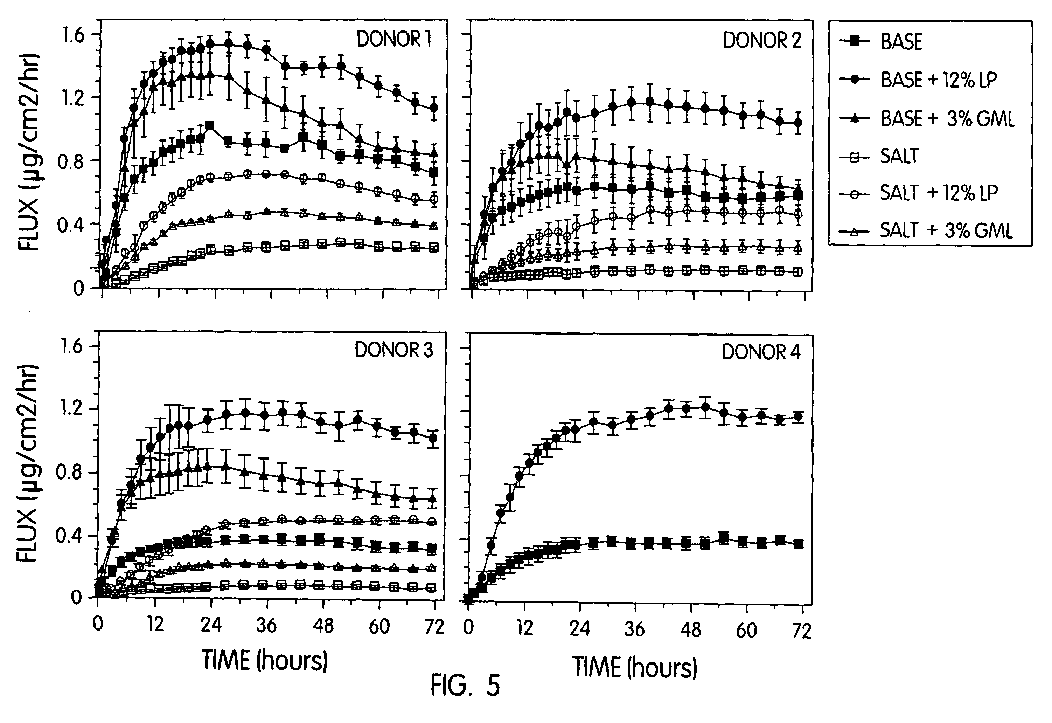

A typical transdermal flux versus time profile obtained across human epidermis from abdominal skin of a specific skin donor for all six adhesive matrix systems is shown in Figure 5. Systems with formulations containing the drug as the base displayed higher drug flux than those with the HCl salt. In addition, systems with formulations containing lauryl pyroglutamate consistently exhibited higher drug flux than formulations with glycerol monolaurate for formulations with either the drug base or salt.

### EXAMPLE 2

Automated permeation cells used for passive permeation studies were also used for the assessment of electrotransport drug flux. The donor compartment contained the silver anode while the receptor compartment contained the silver chloride cathode. The electrodes were connected to a Maccor power source. An aqueous donor solution containing about 15 mg/mL of the drug HCl salt was utilized. The receptor solution was Dulbecco's phosphate buffered saline (0.015 M NaCl) pH 7.4. The first 16 hours served as a zero current passive phase after which the power source was turned on. The electrotransport phase during which a current of 0.2 mA was supplied (to result in a current density of 0.1 mA/cm²) lasted 24 hours. Receptor solution was automatically collected every 4 hours during the passive phase and every hour during the electrotransport phase, and analyzed for drug by HPLC. Flux, as µg/cm²), was calculated, as was the average steady-state flux, which included data points where delivery had reached a nearly constant value.

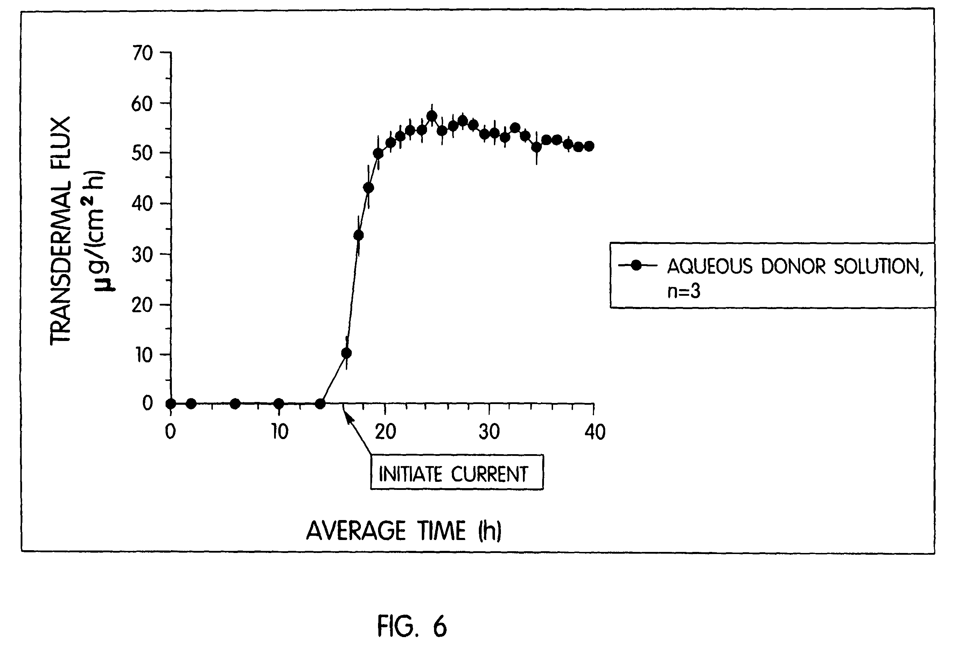

Figure 6 shows the flux profile obtained in this experiment. During the initial 16 hours of the zero-current passive phase, a flux value averaging about 0.02 µg/Cm²h was obtained. Initiation of electrotransport (current 0.2 mA and current density of 0.1 mA/cm²) resulted in a rapid enhancement of drug flux reaching an average steady-state value of 53 ± 2.5 µg/cm²h. Rapid achievement of CNS 5161 delivery via electrotransport suggests that patient-controlled on-demand dosing of this compound is possible.

## Claims

1. A device for the transdermal administration of a therapeutic agent at a therapeutically effective rate, the device comprising:
(a) a reservoir containing a therapeutic agent comprising a compound selected from the group consisting of N-(2,5-disubstituted phenyl)-N'-(3-substituted phenyl)-N'-methyl guanidines and pharmaceutically acceptable salts thereof;
(b) a backing behind the body distal surface of the reservoir; and
(c) means for maintaining the reservoir in therapeutic agent transmitting relation with a body surface or membrane, wherein a therapeutically effective amount of guanidine is delivered at a therapeutically effective rate during an administration period in order to achieve and maintain therapeutic blood or plasma levels throughout a substantial portion of the administration period.

2. A device according to claim 1 wherein the reservoir further contains a permeation enhancing amount of a permeation enhancer.

3. A device according to claim 2 wherein the permeation enhancer is selected from the group consisting of lauryl pyroglutamate, fatty acids, monoglycerides of fatty acids, lactate esters of fatty acids, acyl lactylates, esters of fatty acids having from 10 to 20 carbon atoms, alkyl laurates, monoalkyl ethers of polyethyleneglycol and their alkyl or aryl carboxylic acid esters, carboxymethyl ethers, lower C₁₋₄ alcohols, and mixtures thereof.

4. A device according to claim 3 wherein the permeation enhancer comprises glycerol monolaurate, glycerol monooleate, glycerol monocaprate, glycerol monocaprylate, glycerol monolinoleate, lauryl lactate, cetyl lactate, myristyl lactate, caproyl lactylic acid, isopropyl myristate, ethyl palmitate, methyl laurate, dimethyl lauramide, lauryl acetate, polyethylene glycol-4 lauryl ether (Laureth-4), polyethylene glycol-2 lauryl ether (Laureth-2), polyethylene glycol monolaurate, myristyl sarcosine, myreth-3, isopropanol or ethanol.

5. A device according to claim 2, 3 or 4 wherein the permeation enhancer is lauryl pyroglutamate.

6. A device according to any preceding claim wherein the reservoir further includes a carrier.

7. A device according to claim 6 wherein the carrier is polymeric.

8. A device according to claim 6 or 7 wherein the reservoir comprises:
(a) 1 to 30% by weight of the therapeutic agent;
(b) 0 to 50% by weight of the permeation enhancer; and
(c) 30 to 90% by weight of the polymeric carrier;
or
(a) 1 to 25% by weight of the therapeutic agent;
(b) 1 to 30% by weight of the permeation enhancer; and
(c) 30 to 90% by weight of the polymeric carrier;
or
(a) 1 to 15% by weight N-(2-chloro-5-(methylthio)phenyl)-N'-(3-methylthio)phenyl)-N'-methyl guanidine;
(b) 4 to 20% by weight lauryl pyroglutamate; and
(c) 30 to 90% by weight of the polymeric carrier.

9. A device according to any preceding claim wherein the reservoir comprises a pressure sensitive adhesive which further acts as the means for maintaining the reservoir in guanidine transmitting relation with a body surface or membrane.

10. A device according to any preceding claim for administering 1 - 6 mg/day of guanidine.

11. A device according to claim 10 for administering 2 - 3 mg/day of guanidine.

12. A device according to any preceding claim for administering guanidine at a rate of 20 - 5500 µg/hr.

13. A device according to claim 12 for administering guanidine at a rate of 60 - 600 µg/hr.

14. A device according to any preceding claim for administering guanidine for a period of 24 -72 hours.

## Patentansprüche

1. Vorrichtung zur transdermalen Verabreichung eines therapeutischen Mittels mit einer therapeutisch wirksamen Rate, wobei die Vorrichtung umfasst
(a) ein Reservoir, das ein therapeutisches Mittel enthält, umfassend eine Verbindung, die aus der Gruppe ausgewählt ist, die aus N-(2,5-disubstituiertes Phenyl)-N'-(3-substituiertes Phenyl)-N'-methylguanidinen und pharmazeutisch geeigneten Salzen davon besteht;
(b) eine Stutzschicht hinter der vom Körper abgewandten Seite des Reservoirs; und
(c) eine Möglichkeit, das Reservoir in einer das therapeutische Mittel übertragenden Beziehung mit einer Körperoberfläche oder Membran zu halten, wobei eine therapeutisch wirksame Menge Guanidin mit einer therapeutisch wirksamen Rate während eines Verabreichungszeitraums abgegeben wird, um therapeutische Blut- oder Plasmalevel über einen wesentlichen Teil des Verabreichungszeitraums zu erzielen und beizubehalten.

2. Vorrichtung nach Anspruch 1, worin das Reservoir weiterhin eine permeationsverstärkende Menge eines Permeationsverstarkers enthält.

3. Vorrichtung nach Anspruch 2, worin der Permeationsverstärker aus der Gruppe ausgewählt ist, die aus Laurylpyroglutamat, Fettsäuren, Monoglyceriden von Fettsäuren, Lactatestem von Fettsäuren, Acyllactylaten, Estern von Fettsäuren mit 10 bis 20 Kohlenstoffatomen, Alkyllauraten, Monoalkylethem von Polyethylenglycol und ihren Alkyl- oder Arylcarbonsäureestem, Carboxymethylethern, niederen C₁₋₄-Alkoholen und deren Gemischen besteht.

4. Vorrichtung nach Anspruch 3, worin der Permeationsverstäfker Glycerinmonolaurat, Glycerinmonooleat Glycerinnmonocaprat, Glycerinmonocaprylat, Glycerinmonolinoleat Lauryllactat, Cetyllactat, Myristyllactat, Caproyllactylsäure, Isopropylmyristat, Ethylpalmitat, Methyllaurat, Dimethyllauramid, Laurylacetat, Polyethylenglycol-4-laurylether (Laureth-4), Polyethylenglycol-2-laurylether (Laureth-2), Polyethylenglycolmonolaurat, Myristylsarcosin, Myreth-3, Isopropanol oder Ethanol besteht. :

5. Vorrichtung nach den Ansprüchen: 2, 3 oder 4, worin der Permeationsverstärker Laurylpyroglutamat ist.

6. Vorrichtung nach einem der vorhergehenden Ansprüche, worin das Reservoir weiterhin einen Träger umfasst.

7. Vorrichtung nach Anspruch 6, worin der Träger polymer ist.

8. Vorrichtung nach den Ansprüchen 6 oder 7, worin das Reservoir umfasst:
(a) 1 bis 30 Gew.-% des therapeutischen Mittels;
(b) 0 bis 50 Gew.-% des Permeationsverstärkers; und
(c) 30 bis 90 Gew.-% des polymeren Trägers;
oder
(a) 1 bis 25 Gew.-% des therapeutischen Mittels;
(b) 1 bis 30 Gew.-% des Permeationsverstärkers; und
(c) 30 bis 90 Gew.-% des polymeren Trägers;
oder
(a) 1 bis 15 Gew:-% N-(2-Chlor-5-(methylthio)phenyl)-N'-(3-methylthio)phenyl)-N'-methylguanidin;
(b) 4 bis 20 Gew.-% Laurylpyroglutamat; und
(c) 30 bis 90 Gew.-% des polymeren Trägers.

9. Vorrichtung nach einem der vorhergehenden Ansprüche, worin das Reservoir einen druckempfindlichen Klebstoff umfasst, der darüber hinaus als Möglichkeit wirkt, um das Reservoir in einer Guanidin-übertragenden Beziehung mit einer Körperoberfläche oder Membran zu halten.

10. Vorrichtung nach einem der vorhergehenden Ansprüche zur Verabreichung von 1 - 6 mg/Tag Guanidin.

11. Vorrichtung nach Anspruch 10 zur Verabreichung von 2 - 3 mg/Tag Guanidin.

12. Vorrichtung nach einem der vorhergehenden Ansprüche zur Verabreichung von Guanidin mit einer Rate von 20 - 5500 µg/h.

13. Vorrichtung nach Anspruch 12 zur Verabreichung von Guanidin mit einer Rate von 60 - 600 µg/h.

14. Vorrichtung nach einem der vorhergehenden Ansprüche zur Verabreichung von Guanidin über einen Zeitraum von 24 - 72 Stunden.

## Revendications

1. Dispositif pour l'administration transdermique d'un agent thérapeutique à une vitesse thérapeutiquement efficace, le dispositif comprenant :
(a) un réservoir contenant un agent thérapeutique comprenant un composé choisi dans le groupe constitué par le N-(2,5- disubstitué phényl)-N'-(3- substitué phényl)-N'-méthyl guanidines et les sels pharmaceutiquement acceptables de celles-ci ;
(b) un support derrière la surface distale par rapport au corps du réservoir ; et
(c) un moyen pour maintenir le réservoir en relation de transmission d'agent thérapeutique avec une surface ou membrane corporelle,
dans lequel une quantité thérapeutiquement efficace de guanidine est administrée à une vitesse thérapeutiquement efficace pendant une période d'administration de façon à parvenir et à maintenir des niveaux thérapeutiques dans le sang ou dans le plasma tout au cours d'une partie substantielle de la péroide d'administration.

2. Dispositif selon la revendication 1, dans lequel le réservoir contient en outre un agent augmentant la pénétration d'un agent augmentant la pénétration.

3. Dispositif selon la revendication 2, dans lequel l'agent augmentant la pénétration est choisi dans le groupe constitué par le pyroglutamate de lauryle, les acides gras, les monoglycérides d'acides gras, les esters lactates d'acides gras, les lactylates d'acyle, les esters d'acides gras ayant de 10 à 20 atomes de carbone, les laurates d'alkyle, les éthers monoalkyliques de polyéthylène glycol et leurs esters d'acides alkyl ou aryl carboxyliques, les carboxyméthyl éthers, les alcools inférieurs en C₁₋₄, et leurs mélanges.

4. Dispositif selon la revendication 3, dans lequel l'agent augmentant la pénétration comprend le monolaurate de glycérol, le monooléate de glycérol, le monocaprate de glycérol, le monocaprylate de glycérol, le monolinoléate de glycérol, le lactate de lauryle, le lactate de cétyle, le lactate de myristyle, l'acide caproyl lactylique, le myristate d'isopropyle, le palmitate d'éthyle, le laurate de méthyle, le diméthyl lauramide, l'acétate de lauryle, le polyéthylène glycol-4 lauryl éther (Laureth-4), le polyéthylène glycol-2 lauryl éther (Laureth-2), le monolaurate de polyéthylène glycol, la myristyl sarcosine, le myreth-3, l'isopropanol ou l'éthanol.

5. Dispositif selon l'une des revendications 2, 3 ou 4, dans lequel l'agent augmentant la pénétration est le pyroglutamate de lauryle.

6. Dispositif selon l'une quelconque des revendications précédentes, dans lequel le réservoir comprend en outre un support.

7. Dispositif selon la revendication 6, dans lequel le support est polymère.

8. Dispositif selon l'une des revendications 6 ou 7, dans lequel le réservoir comprend :
(a) 1 à 30% en poids de l'agent thérapeutique ;
(b) 0 à 50% en poids de l'agent augmentant la pénétration ; et
(c) 30 à 90% en poids du support polymère ;
ou
(a) 1 à 25% en poids de l'agent thérapeutique ;
(b) 1 à 30% en poids de l'agent augmentant la pénétration ; et
(c) 30 à 90% en poids du support polymère ;
ou
(a) 1 à 15% en poids de N-(2-chloro-5-(méthylthio)phényl)-N'-(3-méthylthio)phényl-N'-méthyl guanidine ;
(b) 4 à 20% en poids de pyroglutamate de lauryle ; et
(c) 30 à 90% en poids du support polymère.

9. Dispositif selon l'une quelconque des revendications précédentes, dans lequel le réservoir comprend un adhésif sensible à la pression qui agit en outre comme moyen pour maintenir le réservoir en relation de transmission de guanidine avec une surface ou membrane corporelle.

10. Dispositif selon l'une quelconque des revendications précédentes pour l'administration de 1-6 mg/jour de guanidine.

11. Dispositif selon la revendication 10 pour l'administration de 2-3 mg/jour de guanidine.

12. Dispositif selon l'une quelconque des revendications précédentes pour l'administration de guanidine à une vitesse de 20-5500 µg/h.

13. Dispositif selon la revendication 12 pour l'administration de guanidine à une vitesse de 60-600 µg/h.

14. Dispositif selon l'une quelconque des revendications précédentes pour l'administration de guanidine pendant une période de temps de 24-72 heures.
